# EUROPEAN PATENT SPECIFICATION

(11) **EP 0 268 309 B1**
(45) Date of publication and mention of the grant of the patent: **11.12.1996**
(21) Application number: 87201784.3
(22) Date of filing: 18.09.1987
(51) Int. Cl.: A61K 31/505

(54) **Serotonin antagonists for treating wounds**
Serotonin-Antagonisten zur Behandlung von Wunden
Antagonistes de la sérotonine pour le traitement des plaies

(30) Priority: 22.09.1986 US 909991
(43) Date of publication of application: 25.05.1988
(73) Proprietor: JANSSEN PHARMACEUTICA N.V., B-2340 Beerse (BE)
(72) Inventor: Ooms, Leo Alfons André, B-2152 Wechelderzande Lille (BE); Degryse, Anne-Dominique Alice Yvette, B-2410 Herentals (BE)

(56) References cited:
- GB-A- 2 125 398
- Trends in Pharmacological Sciences, Receptor Nomenclature Supplement 1991, page 17
- Neuropharmacology, 25(6) 1986, pages 563-576
- J. Med. Chem. 30(1) January 1987, pages 1-12
- Neuropharmacology 23(12B) December 1984, pages 1503-1510
- Neuropsychopharmacology 3(5-6) October-December 1990, pages 371-383
- Topics in Pharmaceutical Sciences 1989, chapter 6, pages 69-82
- Progress in Drug Research, 30, 1986, pages 365-389 and 446-471

## Description

### Background of the Invention:

A rapid and smooth healing of wounds of various nature is generally considered an important goal to achieve. This is mainly due to the fact that the risk of infections and of concomittant complications tends to increase with the duration and complexity of the healing process.

Up until now, quite a number of preparations have been developed to improve the wound-healing process, said preparations containing various active ingredients such as, for example, disinfectants, antibiotics, anti-inflammatory agents, etc..

Quite unexpectedly, it now has been found that the topical treatment of wounds with agents having serotonin-antagonistic properties enhances and improves the wound-healing process.

Praxis, Vol. 58, No. 11, 18th March 1969, pp. 337-342, by H. Helmig, discusses the effect of the serotonin antagonist benzpiperylon on the course of wound-healing. The only significant effect ascertained is a slight reduction or retardation in collagen formation. No apparent effects are noted as regards leukocyte infiltration, fibroblast proliferation or epidermal regeneration. Wound-healing is reportedly not inhibited.

In Br. J. Pharmac., Vol. 59, No. 4, pp. 591-597, 1977 by E. Law and A.J. Lewis discloses that antagonists of 5-hydroxytryptamine (5-HT) reduces erythema of the skin of rats exposed to U.V. light. The reported results indicate that in the mediation of the erythema reaction 5-HT may perhaps play a minor role.

Neuropharmacology, Vol. 23, No. 12B, pp. 1503-1510, 1984 corresponds to a critical review of peripheral 5-hydroxytryptamine receptors and their classification.

Progress in Drug Research, Vol. 30, pp. 365-389, pp. 446-471, 1986 describes recent advances in central 5-hydroxytryptamine receptor agonists and antagonists. This review offers a discussion of CNS applications of said compounds.

GB-2,125,398 describes bridged piperidyl esters and amides as serotonin M antagonists, i.e. 5-HT3 antagonists, with utility in treatment of analgesia, headaches and arrhythmias.

### Description of the Invention:

The present invention is concerned with the use for the manufacture of a medicament for treating wounds of a compound having serotonin antagonistic properties, said compound being represented by the general formula 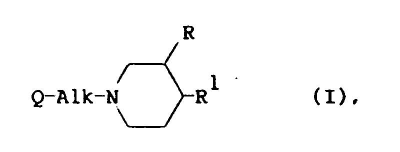 the pharmaceutically acceptable acid-addition salts thereof and the stereoisomeric forms thereof; wherein
R is hydrogen or C₁₋₆alkyl;
Alk is C₁₋₄alkanediyl;
Q is a radical of formula 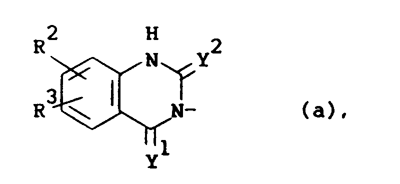 wherein Y¹ and Y² are each independently O or S;
R² is hydrogen, halo, C₁₋₆alkyl, C₁₋₆alkyloxy, CF₃, NO₂, cyano, hydroxy, (C₁₋₁₀alkylcarbonyl)oxy, amino, mono- and di(C₁₋₆alkyl)amino, (C₁₋₁₀alkylcarbonyl)amino, phenylmethoxy or azido;
R³ is hydrogen or halo; or
Q is a radical of formula 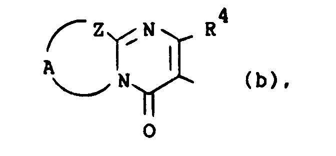 wherein R⁴ is hydrogen or C₁₋₆alkyl;
Z is -S-, -CH₂- or -CR⁵=CR⁶-; said R⁵ and R⁶ being each independently hydrogen or C₁₋₆alkyl; and A is a bivalent radical -CH₂-CH₂-, -CH₂-CH₂-CH₂- or -CR⁷=CR⁸-, said R⁷ and R⁸ being each independently hydrogen, halo, amino or C₁₋₆alkyl;
R¹ is a radical of formula

-X-Ar (c),

wherein Ar is phenyl or substituted phenyl, said substituted phenyl bearing an amino group and/or 1, 2 or 3 halo atoms; and
wherein X is 〉C=O, 〉CH-OH, 〉CH-O-C(O)-R⁹, 〉CH₂, 〉C(OC₁₋₆alkyl)₂, 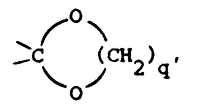 〉C=N-OH or 〉C=N-NH₂;
said R⁹ being hydrogen or C₁₋₆ alkyl and
said q being the integer 2 or 3; or
R¹ is a radical of formula 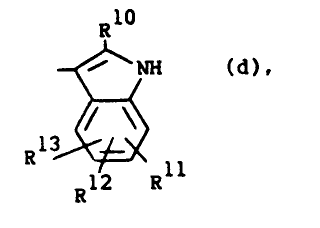 wherein R¹⁰ is hydrogen or C₁₋₆alkyl; R¹¹, R¹² and R¹³ are each independently hydrogen or halo;
or R¹ is a radical of formula 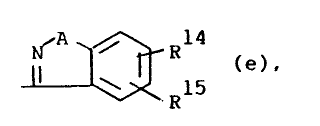 wherein A is O or S; R¹⁴ and R¹⁵ are each independently hydrogen, halo, hydroxy, C₁₋₆alkyloxy or C₁₋₆alkyl.

As used in the foregoing definitions the term halo is generic to fluoro, chloro, bromo and iodo; the term "C₁₋₆alkyl" is meant to include straight and branch chained saturated hydrocarbon radicals having from 1 to 6 carbon atoms such as, for example, methyl, ethyl, 1-methylethyl, 1,1-dimethylethyl, propyl, 2-methylpropyl, butyl, pentyl, hexyl; "C₁₋₁₀alkyl" is meant to include C₁₋₆alkyl radicals, as defined hereinabove, and the higher homologs thereof having from 7 to 10 carbon atoms; and "C₁₋₄alkanediyl" is meant to include bivalent straight or branch chained alkanediyl radicals having from 1 to 4 carbon atoms.

Preferred compounds of formula (I) for use in the manufacture of a medicament for treating wounds are those wherein R¹ is a radical of formula (c).

Particularly preferred compounds of formula (I) for use in the manufacture of a medicament for treating wounds are those preferred compounds of formula (I) wherein Q is a radical of formula (a) wherein Y¹ and Y² are both oxygen atoms and R² and R³ are both hydrogen; or wherein Q is a radical of formula (b), wherein R⁴ is methyl, Z is -CR⁵=CR⁶- wherein R⁵ and R⁶ are independently hydrogen or methyl, A is -CR⁷=CR⁸- wherein R⁷ and R⁸ are independently hydrogen or methyl; and wherein in the radical (c) X is 〉C=O and Ar is halo substituted phenyl.

The most preferred compounds for use in the manufacture of a medicament for treating wounds are selected from the group consisting of 3-[2-[4-(4-fluoro-benzoyl)-1-piperidinyl]ethyl]-2,4(1H,3H-quinazolinedione which compound is generically designated as ketanserin, and 3-[2-[4-(4-fluorobenzoyl)-1-piperidinyl]ethyl)-2,7-dimethyl-4H-pyrido-[1,2-a]pyrimidin-4-one and the pharmaceutically acceptable acid- addition salts thereof.

The compounds of formula (I) can be used as such or in their acid-addition salt form. The latter can conveniently be obtained by treating the base-form with appropriate acids, such as, for example, inorganic acids, such as hydrohalic acid, e.g. hydrochloric, hydrobromic, and sulfuric acid, nitric acid, phosphoric acid; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic.

The compounds of formula (I) preferably used in the manufacture of a medicament for treating wounds are known serotonin antagonists and their preparation as well as their pharmacological properties have been described in U.S. Pat. Nos. 4,335,127, 4,342,870, 4,443,451 and in the Published Eur. Pat. Appl. No. 0,184,258.

The serotonin antagonistic compounds for use in the manufacture of a medicament for treating wounds are most preferably applied in the form of appropriate compositions, in particular compositions usually employed for topically administering drugs. Said compositions may take a wide variety of forms such as, for example, solid forms, e.g. powders; liquid forms, e.g. solutions or suspensions in aqueous or oily mediums; semi-liquid formulations, e.g. creams, jellies, pastes, ointments, salves. Said compositions contain the active ingredient and a wound-acceptable carrier. If desired, further ingredients may be incorporated in the compositions, e.g. anti-inflammatory agents, disinfectants, antibiotics, etc... Moreover, use may be made of wound-covers, e.g. plasters, bandages, dressings, gauze pads, containing an appropriate wound healing composition. In particular use may be made of plasters, bandages, dressings, gauze pads which have been impregnated or sprinkled with a liquid formulation containing the wound healing agent, e.g. with an aseptic aqueous solution, and used as such or, after evaporation of the liquid carrier, in dry form. Or said plasters, bandages, dressings, aseptic gauzes may have been strewn with a powdery solid composition or smeared, covered or coated with a semi-liquid composition. Preferred compositions are creams.

The medicaments of the present invention are applicable to either humans or animals. As wounds which can be treated with the present wound healing medicaments, there may be mentioned all kinds of wounds caused intentionally or not intentionally, the latter being wounds caused, for example, by accidents of various kind, e.g. burning wounds, cutting wounds, puncture wounds, etc..., the former being wounds caused by surgical operations, either by major or minor, human or veterinary surgery, also including transplantation wounds, e.g. wounds originating from skin transplantation. In particular, the medicaments according to the present invention are useful in the treatment of burning wounds and bedsore wounds
An advantage of the medicaments of the present invention resides in the fact that, besides having a beneficial effect on the healing process, there is also an effect on the infections often occuring during the healing process. Wounds treated with the medicaments of this invention will be less subject or show an increased resistance to infections. Also, if they occur, infections will be less intense and can be overcome or healed more rapidly.
The medicaments according to the present invention may be advantageously used in the treatment of ulcerating wounds and are particularly applicable in the treatment of chronic ulcerating wounds. The latter renders the present invention even more useful since up until now, there is no completely satisfying treatment of such wounds.

In a further aspect of the present invention, there is provided a wound-healing composition containing as active ingredient a compound of formula (I) as defined hereinabove, and a wound-acceptable carrier, and, if desired one or more further active ingredients such as disinfectants, antibiotics and anti-inflammatory agents. Said wound-healing compositions can be prepared following methods generally employed in the art of pharmaceutical formulation, e.g., for preparing powders by thoroughly grinding and mixing the components; for solutions by dissolving the active ingredient in the liquid medium by shaking, stirring, if desirable, at higher temperatures; for semi-liquid formulations by dispersing the active ingredient in the semi-liquid carrier.

The following examples are intented to illustrate the scope of the present invention in all its aspects. As used in the following examples Pluronic L 35® is a trademark for a block-copolymer containing polyoxyethylene and polyoxypropylene blocks.

### A. Composition examples

### Example 1

| Placebo (formulation 1): | |
|---|---|
| propyleneglycol | 100.0 mg |
| hydroxypropylmethylcellulose | 15.5 mg |
| water | 884.5 mg |

The ingredients are mixed under intense stirring.

### Example 2

| 3-[2-[4-(4-fluorobenzoyl)-1-piperidinyl]ethyl]-2,4(1H,3H-quinazolinedione (ketanserin) 0.25% (formulation 2): | |
|---|---|
| 3-[2-[4-(4-fluorobenzoyl)-1-piperidinyl]ethyl]-2,4(1H,3H-quinazolinedione tartrate | 3.46 mg |
| propyleneglycol | 99.0 mg |
| hydroxypropylmethylcellulose | 15.6 mg |
| water | 881.9 mg |

The active ingredient ketanserin tartrate is added to the water under vigorous stirring, whereupon the other ingredients are added.

### Example 3

| 3-[2-[4-(4-fluorobenzoyl)-1-piperidinyl]ethyl]-2,7-dimethyl-4H-pyrido-[1,2-a]pyrimidin-4-one 0.25% (formulation 3): | |
|---|---|
| 3-[2-[4-(4-fluorobenzoyl)-1-piperidinyl]ethyl]-2,7-dimethyl 4H-pyrido[1,2-a]pyrimidin-4-one | 2.5 mg |
| propyleneglycol | 99.0 mg |
| hydroxypropylmethylcellulose | 15.6 mg |
| water | 881.9 mg |

### Example 4

| | |
|---|---|
| Ketanserin microfine | 2% |
| Pluronic L 35® | 90.5% |
| Glycerol monostearate | 7.5% |

### Method of Preparation

Glycerol monostearate and Pluronic L 35® are introduced into a doublewall jacketed vessel and are heated to approximately 65°C. Stirring is applied until a homogeneous, clear liquid is obtained. This clear liquid is cooled to approximately 40°C, while gently stirring. Hereupon a slightly viscous, unctuous mass is formed. Subsequently, while homogenizing the mixture, ketanserin is sucked into the ointment by evacuating the vessel. Homogenizing is applied until ketanserin is evenly distributed in the ointment. The ointment is further cooled to 20-25°C while gently stirring.

### Example 5

| | |
|---|---|
| Ketanserin microfine | 2% |
| Polyethyleneglycol 400 | 78% |
| Polyethyleneglycol 4000 | 20% |

### Method of Preparation

Polyethyleneglycol 4000 and polyethyleneglycol 400 are introduced into a doublewall jacketed vessel and are heated to approximately 65°C. Stirring is applied until a homogeneous, clear liquid is obtained. This clear liquid is cooled to approximately 40°C, while gently stirring. Hereupon a slightly viscous, unctuous mass is formed. Subsequently, while homogenizing the mixture, ketanserin powder is sucked into the ointment by evacuating the vessel. Homogenizing is applied until ketanserin is evenly distributed in the ointment. The ointment is further cooled to 20-25°C, while gently stirring.

### B. Pharmacological Examples

The useful wound-healing properties of the compounds of formula (I) can be demonstrated by the following experiments.

### Example 6

### Materials and Methods

40 Male Wistar rats (mean body weight 150 g) were used. They were divided into four groups each consisting of 10 animals: control group, placebo group, formulation 2 group, and formulation 3 group. After ether anaesthesia, the flanks of the animals were clipped and disinfected. Wounds were made as follows: through a skinfold of the thoracic skin two circular skin flaps (1 cm diameter) were excised using a punch.

A once daily treatment was started one day after surgery. In all groups, wounds were dipped with a gauze pad soaked with a disinfectant. Thereafter either a dry gauze pad (control group) or a gauze pad dipped in the test formulation (formulations 1(placebo), 2 or 3) was then wrapped around the body and fixed with an adhesive tape. Blind scoring consisted of the recording of the presence or absence of complete healing of wounds 10 days after the start of the experiment.

### Results

**Table 1**

| Wound healing after 10 days | | |
|---|---|---|
| Treatment group | No. of animals | No. of healed wounds vs. total |
| control | 10 | 5/20 |
| formulation 1 | 10 | 10/20 |
| formulation 2 | 10 | 15/20 |
| formulation 3 | 10 | 14/20 |

Significantly faster wound-healing was found for wounds treated with formulations 2 and 3 compared to control wounds and two placebo-treated wounds (formulation 1).

## Claims (Claims for the following Contracting State(s): AT, BE, FR, DE, GB, NL, IT, LI, LU, SE, CH)

1. The use for the manufacture of a medicament for treating wounds of a compound having the formula 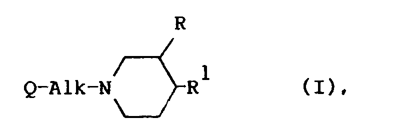 a pharmaceutically acceptable acid-addition salt thereof or a stereoisomeric form thereof; wherein
R is hydrogen or C₁₋₆alkyl;
Alk is C₁₋₄alkanediyl;
Q is a radical of formula 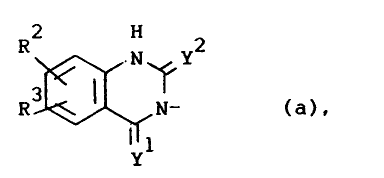 wherein Y¹ and Y² are each independently O or S;
R² is hydrogen, halo, C₁₋₆alkyl, C₁₋₆alkyloxy, CF₃, NO₂, cyano, hydroxy, (C₁₋₁₀alkylcarbonyl)oxy, amino, mono- and di(C₁₋₆alkyl)amino, (C₁₋₁₀alkylcarbonyl)amino, phenylmethoxy or azido;
R³ is hydrogen or halo; or
Q is a radical of formula 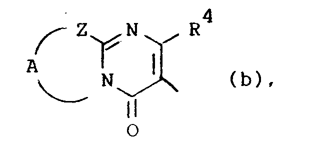 wherein R⁴ is hydrogen or C₁₋₆alkyl;
Z is -S-, -CH₂- or -CR⁵=CR⁶-; said R⁵ and R⁶ being each independently hydrogen or C₁₋₆alkyl; and A is a bivalent radical -CH₂-CH₂-, -CH₂-CH₂-CH₂- or -CR⁷=CR⁸-, said R⁷ and R⁸ being each independently hydrogen, halo, amino or C₁₋₆alkyl;
R¹ is a radical of formula
-X-Ar (c),
wherein Ar is phenyl or substituted phenyl, said substituted phenyl bearing an amino group and/or 1, 2 or 3 halo atoms; and
wherein X is 〉C=O, 〉CH-OH, 〉CH-O-C(O)-R⁹, 〉CH₂, 〉C(OC₁₋₆alkyl)₂, 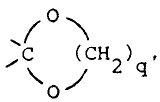 〉C=N-OH or 〉C=N-NH₂;
said R⁹ being hydrogen or C₁₋₆ alkyl and
said q being the integer 2 or 3; or
R¹ is a radical of formula 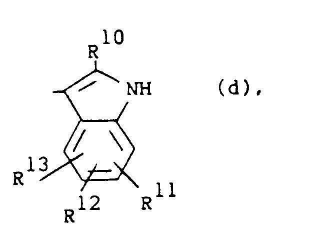 wherein R¹⁰ is hydrogen or C₁₋₆alkyl; R¹¹, R¹² and R¹³ are each independently hydrogen or halo;
or R¹ is a radical of formula 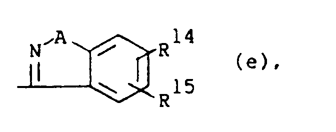 wherein A is O or S; R¹⁴ and R¹⁵ are each independently hydrogen, halo, hydroxy, C₁₋₆alkyloxy or C₁₋₆alkyl.

2. The use according to claim 1 wherein R¹ is a radical of formula (c).

3. The use according to claim 2 wherein Q is a radical of formula (a) wherein Y¹ and Y² are both oxygen atoms and R² and R³ are both hydrogen; or wherein Q is a radical of formula (b), wherein R⁴ is methyl, Z is -CR⁵=CR⁶- wherein R⁵ and R⁶ are independently hydrogen or methyl, A is -CR⁷=CR⁸- wherein R⁷ and R⁸ are independently hydrogen or methyl; and wherein in the radical (c) X is 〉C=O and Ar is halo substituted phenyl.

4. The use according to claim 1 wherein the compound is 3-[2-[4-(4-fluorobenzoyl)-1-piperidinyl]ethyl]-2,4(1H,3H-quinazolinedione or 3-[2-[4-(4-fluorobenzoyl)-1-piperidinyl]ethyl]-2,7-dimethyl-4H-pyrido-[1,2-a]pyrimidin-4-one.

5. A wound-healing composition comprising a wound-acceptable carrier and as active ingredient a compound selected from the group of compounds having the formula (I) as defined in any of claims 1 to 4.

6. A composition according to claim 5 which is topically applicable.

7. A composition according to claim 6 which is a powder, an aqueous or oily solution or suspension or a semi-liquid formulation.

8. A composition according to claim 7 which is a cream, a jelly, a paste, an ointment or a salve.

9. A wound-cover containing a wound healing composition as claimed in any of claims 5 to 8.

10. A wound-cover according to claim 9 which is a plaster, bandage, dressing or gauze pad impregnated or sprinkled with a liquid wound-healing composition or strewn with a powdery solid wound-healing composition or smeared, covered or coated with a semi-liquid wound-healing composition.

11. A process for preparing a composition as claimed in any of claims 5 or 8 characterized in that the active ingredient is intimately mixed with the carrier, and if desired, with other active ingredients.

12. A process for preparing a wound-cover according to any of claims 10 or 11 characterized in that a wound healing composition as claimed in any of claims 5 to 8 is contacted with the wound-cover.

## Claims (Claims for the following Contracting State(s): ES, GR)

1. A process for preparing a wound-healing composition comprising a wound-acceptable carrier and as active ingredient a compound selected from the group of compounds having the formula 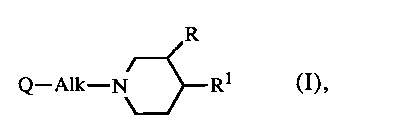 a pharmaceutically acceptable acid addition salt thereof or a stereoisomeric form thereof, wherein
R is hydrogen or C₁₋₆alkyl;
Alk is C₁₋₄alkanediyl;
Q is a radical of formula 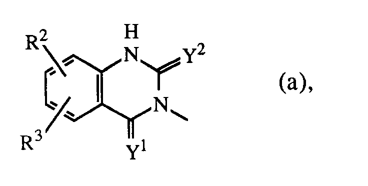 wherein Y¹ and Y² are each independently O or S;
R² is hydrogen, halo, C₁₋₆alkyl, C₁₋₆alkyloxy, trifluoromethyl, nitro, cyano, hydroxy, (C₁₋₁₀alkylcarbonyl)oxy, amino, mono- and di(C₁₋₆alkyl)amino, (C₁₋₁₀alkylcarbonyl)amino, phenylmethoxy or azido;
R³ is hydrogen or halo; or
Q is a radical of formula 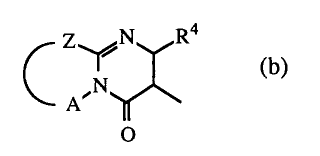 wherein R⁴ is hydrogen or C₁₋₆alkyl;
Z is -S-, -CH₂- or -CR⁵=CR⁶-; said R⁵ and R⁶ being each independently hydrogen or C₁₋₆alkyl; and A is a bivalent radical -CH₂-CH₂-, -CH₂-CH₂-CH₂- or -CR⁷=CR⁸-, said R⁷ and R⁸ being each independently hydrogen, halo, amino or C₁₋₆alkyl;
R¹ is a radical of formula
-X-Ar (c),
wherein Ar is phenyl or substituted phenyl, said substituted phenyl bearing an amino group and/or 1, 2 or 3 halo atoms; and
wherein X is 〉C=O, 〉CH-OH, 〉CH-O-C(O)-R⁹, 〉CH₂, 〉C(OC₁₋₆alkyl)₂, 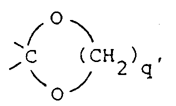 〉C=N-OH or 〉C=N-NH₂;
said R⁹ being hydrogen or C₁₋₆ alkyl and
said q being the integer 2 or 3; or
R¹ is a radical of formula 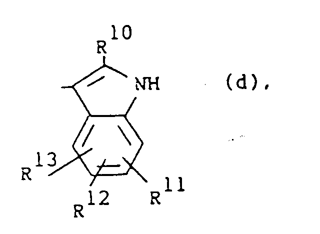 wherein R¹⁰ is hydrogen or C₁₋₆alkyl; R¹¹, R¹² and R¹³ are each independently hydrogen or halo;
or R¹ is a radical of formula 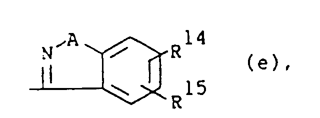 wherein A is O or S; R¹⁴ and R¹⁵ are each independently hydrogen, halo, hydroxy, C₁₋₆alkyloxy or C₁₋₆alkyl,
characterized in that, the active ingredient is intimately mixed with the carrier, and if desired, with other active ingredients.

2. A process according to claim 1 wherein R¹ is a radical of formula (c).

3. A process according to claim 2, wherein Q is a radical of formula (a), wherein Y¹ and Y² are both oxygen atoms and R² and R³ are both hydrogen; or wherein Q is a radical of formula (b), wherein R⁴ is methyl, Z is -CR⁵=CR⁶ wherein R⁵ and R⁶ are independently hydrogen or methyl, A is -CR⁷=CR⁸- wherein R⁷ and R⁸ are independently hydrogen or methyl; and wherein in the radical (c) X is 〉C=O and Ar is halo substituted phenyl.

4. A process according to claim 1 wherein the compound is 3-[2-[4-(4-fluorobenzoyl)-1-piperidinyl]ethyl]-2,4(1H,3H-quinazolinedione or 3-[2-[4-(4-fluorobenzoyl)-1-piperidinyl]ethyl]-2,7-dimethyl-4H-pyrido-[1,2-a]pyrimidin-4-one.

5. A process according to claim 1 wherein the composition is topically applicable.

6. A process according to claim 5 wherein the composition is a powder, an aqueous or oily solution or suspension or a semi-liquid formulation.

7. A process according to claim 6 wherein the composition is a cream, a jellly, a paste, an ointment or a salve.

8. A process for preparing wound-cover containing a wound-healing composition as defined in anyone of claims 1 to 7, characterized in that said wound-healing composition is contacted with the wound-cover.

9. A process according to claim 8 wherein the wound-cover is a plaster, bandage, dressing or gauze pad impregnated or sprinkled with a liquid wound-healing composition or strewn with a powdery solid would-healing composition or smeared, covered or coated with a semi-liquid wound-healing composition.

## Patentansprüche (Patentansprüche für folgende(n) Vertragsstaat(en): AT, BE, FR, DE, GB, NL, IT, LI, LU, SE, CH)

1. Verwendung einer Verbindung der Formel 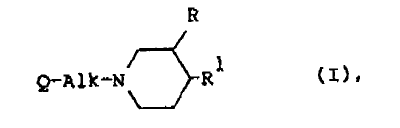 eines ihrer pharmazeutisch unbedenklichen Säureadditionssalze oder einer ihrer stereoisomeren Formen, wobei
R Wasserstoff oder C₁₋₆Alkyl darstellt;
Alk C₁₋₄Alkandiyl darstellt;
Q einen Rest der Formel 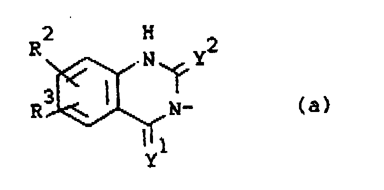 darstellt, wobei Y¹ und Y² jeweils unabhängig O oder S darstellen;
R² Wasserstoff, Halogen, C₁₋₆Alkyl, C₁₋₆Alkyloxy, CF₃, NO₂, Cyan, Hydroxy, (C₁₋₁₀Alkylcarbonyl)oxy, Amino, Mono- und Di-(C₁₋₆Alkyl)amino, (C₁₋₁₀Alkylcarbonyl)amino, Phenylmethoxy oder Azido darstellt;
R³ Wasserstoff oder Halogen darstellt; oder
Q einen Rest der Formel 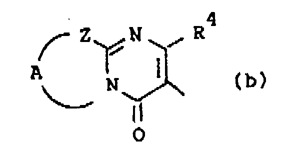 darstellt, wobei R⁴ Wasserstoff oder C₁₋₆Alkyl darstellt;
Z -S-, -CH₂- oder -CR⁵=CR⁶- darstellt; wobei R⁵ und R⁶ jeweils unabhängig Wasserstoff oder C₁₋₆Alkyl darstellen; und A einen zweiwertigen Rest -CH₂-CH₂-, -CH₂-CH₂-CH₂- oder -CR⁷=CR⁸- darstellt, wobei R⁷ und R⁸ jeweils unabhängig Wasserstoff, Halogen, Amino oder C₁₋₆Alkyl darstellen;
R¹ einen Rest der Formel
-X-Ar (c)
darstellt, wobei Ar Phenyl oder substituiertes Phenyl darstellt, wobei das substituierte Phenyl eine Aminogruppe und/oder 1, 2 oder 3 Halogenatome trägt; und
wobei X 〉C=O, 〉CH-OH, 〉CH-O-C(O)-R⁹, 〉CH₂, C(OC₁₋₆Alkyl)₂, 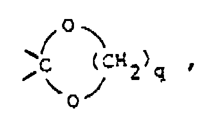 〉C=N-OH oder 〉C=N-NH₂
darstellt;
wobei R⁹ Wasserstoff oder C₁₋₆Alkyl darstellt und
q eine ganze Zahl 2 oder 3 darstellt; oder
R¹ einen Rest der Formel 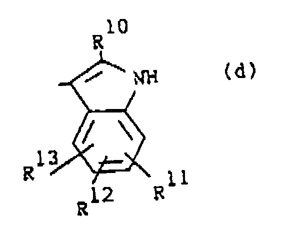 darstellt, wobei R¹⁰ Wasserstoff oder C₁₋₆Alkyl darstellt;
R¹¹, R¹² und R¹³ jeweils unabhängig Wasserstoff oder Halogen darstellen;
oder R¹ einen Rest der Formel 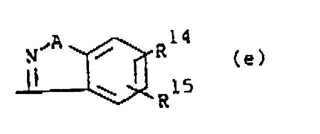 darstellt, wobei A O oder S darstellt; R¹⁴ und R¹⁵ jeweils unabhängig Wasserstoff, Halogen, Hydroxy, C₁₋₆Alkyloxy oder C₁₋₆Alkyl darstellen,
zur Herstellung eines Arzneimittels zur Behandlung von Wunden.

2. Verwendung gemäß Anspruch 1, wobei R¹ einen Rest der Formel (c) darstellt.

3. Verwendung gemäß Anspruch 2, wobei Q einen Rest der Formel (a) darstellt, in welcher sowohl Y¹ als auch Y² Sauerstoffatome darstellen und sowohl R² als auch R³ Wasserstoff darstellen; oder in welcher Q einen Rest der Formel (b) darstellt, in welcher R⁴ Methyl darstellt, Z -CR⁵=CR⁶- darstellt, wobei R⁵ und R⁶ unabhängig Wasserstoff oder Methyl darstellen, A -CR⁷=CR⁸-, wobei R⁷ und R⁸ unabhängig Wasserstoff oder Methyl darstellen, darstellt; und im Rest (c) X 〉C=O sowie Ar halogensubstituiertes Phenyl darstellen.

4. Verwendung gemäß Anspruch 1, wobei es sich bei der Verbindung um 3-[2-[4-(4-Fluorbenzoyl)-1-piperidinyl]ethyl]-2,4(1H,3H-chinazolindion oder 3-[2-[4-(4-Fluorbenzoyl)-1-piperidinyl]ethyl]-2,7-dimethyl-4H-pyrido-[1,2-a]pyrimidin-4-on handelt.

5. Wundheilungsmittel mit einem für Wunden unbedenklichen Träger und, als Wirkstoff, einer Verbindung aus der Verbindungsgruppe der wie in einem der Ansprüche 1 bis 4 definierten Formel (I).

6. Mittel gemäß Anspruch 5, das topisch verabreicht werden kann.

7. Mittel gemäß Anspruch 6 in Form eines Pulvers, einer Lösung oder Suspension in Wasser oder Öl oder einer halbflüssigen Formulierung.

8. Mittel gemäß Anspruch 7 in Form einer Creme, eines Gelees, einer Paste, einer Salbe oder eines Balsams.

9. Wundabdeckung mit einem Wundheilungsmittel gemäß einem der Ansprüche 5 bis 8.

10. Wundabdeckung gemäß Anspruch 9 in Form eines Pflasters, eines Gazekissens, einer Binde oder eines Verbands, das bzw. die bzw. der mit einem flüssigen Wundheilungsmittel getränkt oder beträufelt oder mit einem pulverförmigen festen Wundheilungsmittel bestreut oder mit einem halbflüssigen Wundheilungsmittel bestrichen, bedeckt oder beschichtet ist.

11. Verfahren zur Herstellung eines Mittels gemäß einem der Ansprüche 5 oder 8, dadurch gekennzeichnet, daß man den Wirkstoff mit dem Träger sowie gewünschtenfalls mit weiteren Wirkstoffen innig vermischt.

12. Verfahren zur Herstellung einer Wundabdeckung gemäß einem der Ansprüche 10 oder 11, dadurch gekennzeichnet, daß man ein Wundheilungsmittel gemäß einem der Ansprüche 5 bis 8 mit der Wundabdeckung in Kontakt bringt.

## Patentansprüche (Patentansprüche für folgende(n) Vertragsstaat(en): ES, GR)

1. Verfahren zur Herstellung eines Wundheilungsmittels mit einem für Wunden unbedenklichen Träger und, als Wirkstoff, einer Verbindung aus der Verbindungsgruppe der Formel 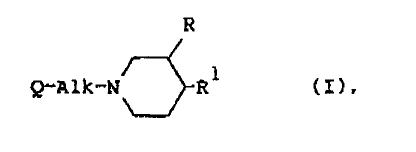 eines ihrer pharmazeutisch unbedenklichen Säureadditionssalze oder einer ihrer stereoisomeren Formen, wobei
R Wasserstoff oder C₁₋₆Alkyl darstellt;
Alk C₁₋₄Alkandiyl darstellt;
Q einen Rest der Formel 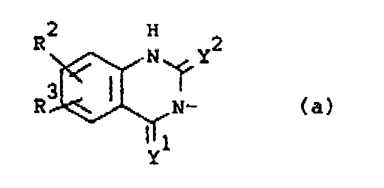 darstellt, wobei Y¹ und Y² jeweils unabhängig O oder S darstellen;
R² Wasserstoff, Halogen, C₁₋₆Alkyl, C₁₋₆Alkyloxy, Trifluoromethyl, Nitro, Cyan, Hydroxy, (C₁₋₁₀Alkylcarbonyl)oxy, Amino, Mono- und Di-(C₁₋₆Alkyl)amino, (C₁₋₁₀Alkylcarbonyl)amino, Phenylmethoxy oder Azido darstellt;
R³ Wasserstoff oder Halogen darstellt; oder
Q einen Rest der Formel 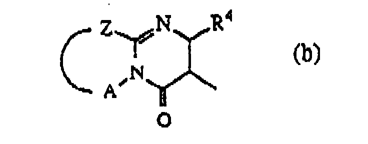 darstellt, wobei R⁴ Wasserstoff oder C₁₋₆Alkyl darstellt;
Z -S-, -CH₂- oder -CR⁵=CR⁶- darstellt; wobei R⁵ und R⁶ jeweils unabhängig Wasserstoff oder C₁₋₆Alkyl darstellen; und A einen zweiwertigen Rest -CH₂-CH₂-, -CH₂-CH₂-CH₂- oder -CR⁷=CR⁸- darstellt, wobei R⁷ und R⁸ jeweils unabhängig Wasserstoff, Halogen, Amino oder C₁₋₆Alkyl darstellen;
R¹ einen Rest der Formel
-X-Ar (c)
darstellt, wobei Ar Phenyl oder substituiertes Phenyl darstellt, wobei das substituierte Phenyl eine Aminogruppe und/oder 1, 2 oder 3 Halogenatome trägt; und
wobei X 〉C=O, 〉CH-OH, 〉CH-O-C(O)-R⁹, 〉CH₂, C(OC₁₋₆Alkyl)₂, 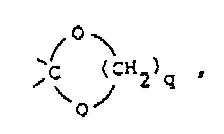 C=N-OH oder C=N-NH₂
darstellt;
wobei R⁹ Wasserstoff oder C₁₋₆Alkyl darstellt und
q eine ganze Zahl 2 oder 3 darstellt; oder
R¹ einen Rest der Formel 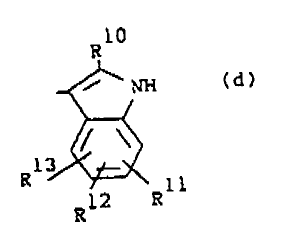 darstellt, wobei R¹⁰ Wasserstoff oder C₁₋₆Alkyl darstellt;
R¹¹, R¹² und R¹³ jeweils unabhängig Wasserstoff oder Halogen darstellen;
oder R¹ einen Rest der Formel 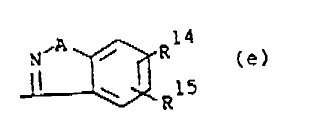 darstellt, wobei A O oder S darstellt; R¹⁴ und R¹⁵ jeweils unabhängig Wasserstoff, Halogen, Hydroxy, C₁₋₆Alkyloxy oder C₁₋₆Alkyl darstellen,
dadurch gekennzeichnet, daß man den Wirkstoff mit dem Träger sowie gewünschtenfalls mit weiteren Wirkstoffen innig vermischt.

2. Verfahren gemäß Anspruch 1, wobei R¹ einen Rest der Formel (c) darstellt.

3. Verfahren gemäß Anspruch 2, wobei Q einen Rest der Formel (a) darstellt, in welcher sowohl Y¹ als auch Y² Sauerstoffatome darstellen und sowohl R² als auch R³ Wasserstoff darstellen; oder in welcher Q einen Rest der Formel (b) darstellt, in welcher R⁴ Methyl darstellt, Z -CR⁵=CR⁶ darstellt, wobei R⁵ und R⁶ unabhängig Wasserstoff oder Methyl darstellen, A -CR⁷=CR⁸-, wobei R⁷ und R⁸ unabhängig Wasserstoff oder Methyl darstellen, darstellt; und im Rest (c) X 〉C=O sowie Ar halogensubstituiertes Phenyl darstellen.

4. Verfahren gemäß Anspruch 1, wobei es sich bei der Verbindung um 3-[2-[4-(4-Fluorbenzoyl)-1-piperidinyl]ethyl]-2,4(1H,3H-chinazolindion oder 3-[2-[4-(4-Fluorbenzoyl)-1-piperidinyl]ethyl]-2,7-dimethyl-4H-pyrido-[1,2-a]pyrimidin-4-on handelt.

5. Verfahren gemäß Anspruch 1, wobei das Mittel topisch verabreicht werden kann.

6. Verfahren gemäß Anspruch 5, wobei das Mittel in Form eines Pulvers, einer Lösung oder Suspension in Wasser oder Öl oder einer halbflüssigen Formulierung vorliegt.

7. Verfahren gemäß Anspruch 6, wobei das Mittel in Form einer Creme, eines Gelees, einer Paste, einer Salbe oder eines Balsams vorliegt.

8. Verfahren zur Herstellung einer Wundabdeckung mit einem Wundheilungsmittel gemäß einem der Ansprüche 1 bis 7, dadurch gekennzeichnet, daß man das Wundheilungsmittel mit der Wundabdeckung in Kontakt bringt.

9. Verfahren gemäß Anspruch 8, wobei die Wundabdeckung in Form eines Pflasters, eines Gazekissens, einer Binde oder eines Verbands, das bzw. die bzw. der mit einem flüssigen Wundheilungsmittel getränkt oder beträufelt oder mit einem pulverförmigen festen Wundheilungsmittel bestreut oder mit einem halbflüssigen Wundheilungsmittel bestrichen, bedeckt oder beschichtet ist, vorliegt.

## Revendications (Revendications pour l'(les) Etat(s) contractant(s) suivant(s): AT, BE, FR, DE, GB, NL, IT, LI, LU, SE, CH)

1. Utilisation, pour la fabrication d'un médicament pour le traitement des plaies, d'un composé de formule 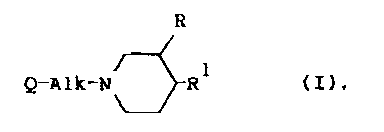 un sel d'addition d'acide pharmaceutiquement acceptable de celui-ci Ou une forme stéréoisomère de celui-ci, caractérisé en ce que
R est hydrogène ou alkyle(C₁₋₆);
Alk est alcane(C₁₋₄)diyle;
Q est un radical de formule 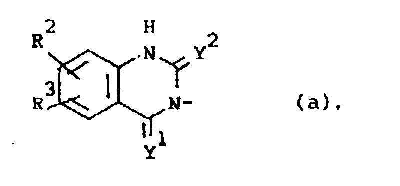 dans laquelle Y¹ et Y² sont chacun indépendamment O ou S;
R² est hydrogène, halogéno, alkyle(C₁₋₆), alkyloxy(C₁₋₆), CF₃, NO₂, cyano, hydroxy, (alkyle(C₁₋₁₀)carbonyl)oxy, amino, mono- et di-alkyle(C₁₋₆)amino, (alkyle(C₁₋₁₀)carbonyl)amino, phénylméthoxy ou azido;
R³ est hydrogène ou halogéno; ou
Q est un radical de formule 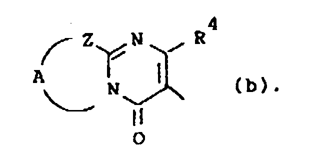 dans laquelle R⁴ est hydrogène ou alkyle(C₁₋₆);
Z est -S-, -CH₂- ou -CR⁵=CR⁶-; lesdits R⁵ et R⁶ étant chacun indépendamment hydrogène ou alkyle(C₁₋₆); et A est un radical bivalent -CH₂-CH₂-, -CH₂-CH₂-CH₂- ou -CR⁷=CR⁸-, lesdits R⁷ et R⁸ étant chacun indépendamment hydrogène, halogéno, amino ou alkyle(C₁₋₆);
R¹ est un radical de formule
-X-Ar (c),
dans laquelle Ar est phényle ou phényle substitué, ledit phényle substitué portant un groupement amino et/ou 1, 2 ou 3 atomes halogéno; et
dans laquelle X est 〉C=O, 〉CH-OH, 〉CH-O-C(O)-R⁹, 〉CH₂, 〉C(Oalkyle(C₁₋₆))₂, 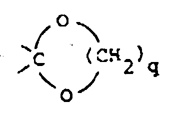 〉C=N-OH or 〉C=N-NH₂;
ledit R⁹ étant hydrogène ou alkyle(C₁₋₆) et
ledit q étant un entier égal à 2 ou 3; ou
R¹ est un radical de formule 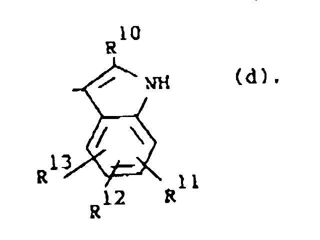 dans laquelle R¹⁰ est hydrogène ou alkyle(C₁₋₆); R¹¹, R¹², et R¹³ sont chacun indépendamment hydrogène ou halogéno;
ou R¹ est un radical de formule 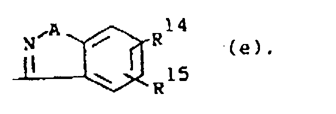 dans laquelle A est O ou S; R¹⁴ et R¹⁵ sont chacun indépendamment hydrogène, halogéno, hydroxy, alkyloxy(C₁₋₆) ou alkyle(C₁₋₆).

2. Utilisation selon la revendication 1, caractérisée en ce que R¹ est un radical de formule (c).

3. Utilisation selon la revendication 2, caractérisée en ce que Q est un radical de formule (a) dans laquelle Y¹ et Y² sont tous deux des atomes d'oxygène et R² et R³ sont tous deux hydrogène; ou en ce que Q est un radical de formule (b), dans laquelle R⁴ est méthyle, Z est -CR⁵=CR⁶- dans laquelle R⁵ et R⁶ sont indépendamment hydrogène ou méthyle, A est -CR⁷=CR⁸- dans laquelle R⁷ et R⁸ sont indépendamment hydrogène ou méthyle; et en ce que dans le radical (c) X est 〉C=O et Ar est phényle susbstitué par halogéno.

4. Utilisation selon la revendication 1, caractérisée en ce que le composé est la 3-[2-[4-(4-fluorobenzoyl)-1-pipéridinyl]éthyl-2,4-(1H, 3H-quinazolinedione ou la 3-[2-[4-(4-fluorobenzoyl)-1-pipéridinyl]éthyl-2,7-diméthyl-4H-pyrido[1,2-a]pyrimidin-4-one.

5. Composition pour la cicatrisation des plaies comprenant un support acceptable pour les plaies et, comme principe actif, un composé choisi parmi le groupe de composés ayant la formule (I) comme définie dans l'une quelconque des revendications 1 à 4.

6. Composition selon la revendication 5, applicable par voie topique.

7. Composition selon la revendication 6, caractérisée en ce qu'il s'agit d'une poudre, d'une solution ou suspension aqueuse ou huileuse ou d'une formulation semi-liquide.

8. Composition selon la revendication 7, caractérisée en ce qu'il s'agit d'une crème, d'une gelée, d'une pâte, d'une pommade ou d'un baume.

9. Couverture de plaie contenant une composition de cicatrisation des plaies selon l'une quelconque des revendications 5 à 8.

10. Couverture de plaie selon la revendication 9, caractérisée en ce qu'il s'agit d'un plâtre, d'un bandage, d'un pansement ou d'un tampon de gaze imprégné ou arrosé d'une composition liquide de cicatrisation des plaies ou sur lequel on a répandu une composition solide pulvérulente de cicatrisation des plaies ou qui est enduit, recouvert ou enrobé d'une composition de cicatrisation des plaies semi-liquide.

11. Procédé de préparation d'une composition selon l'une quelconque des revendications 5 ou 8, caractérisé en ce que le principe actif est mélangé intimement avec le support, et si on le souhaite, avec d'autres principes actifs.

12. Procédé de préparation d'une couverture de plaie selon l'une quelconque des revendications 10 ou 11, caractérisé en ce qu'une composition de cicatrisation des plaies selon l'une quelconque des revendications 5 à 8 est mise en contact avec la couverture de plaie.

## Revendications (Revendications pour l'(les) Etat(s) contractant(s) suivant(s): ES, GR)

1. Procédé de préparation d'une composition pour la cicatrisation des plaies comprenant un support acceptable pour les plaies et, comme principe actif, un composé choisi parmi le groupe de composés ayant la formule 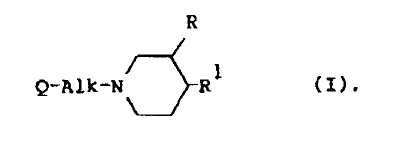 un sel d'addition d'acide pharmaceutiquement acceptable de celui-ci ou une forme stéréoisomère de celui-ci, caractérisé en ce que
R est hydrogène ou alkyle(C₁₋₆);
Alk est alcane(C₁₋₄)diyle;
Q est un radical de formule 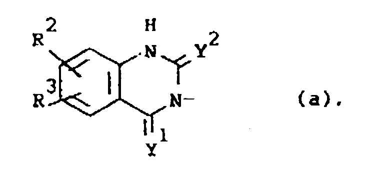 dans laquelle Y¹ et Y² sont chacun indépendamment O ou S;
R² est hydrogène, halogéno, alkyle(C₁₋₆), alkyloxy(C₁₋₆), trifluorométhyle, nitro, cyano, hydroxy, (alkyle(C₁₋₁₀)carbonyl)oxy, amino, mono- et di-alkyle(C₁₋₆)amino, (alkyle(C₁₋₁₀)carbonyl)amino, phénylméthoxy ou azido;
R³ est hydrogène ou halogéno; ou
Q est un radical de formule 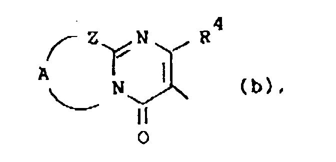 dans laquelle R⁴ est hydrogène Ou alkyle(C₁₋₆);
Z est -S-, -CH₂- ou -CR⁵=CR⁶-; lesdits R⁵ et R⁶ étant chacun indépendamment hydrogène ou alkyle(C₁₋₆); et A est un radical bivalent -CH₂-CH₂-, -CH₂-CH₂-CH₂- ou -CR⁷=CR⁸-, lesdits R⁷ et R⁸ étant chacun indépendamment hydrogène, halogéno, amino ou alkyle(C₁₋₆);
R¹ est un radical de formule
-X-Ar (c),
dans laquelle Ar est phényle ou phényle substitué, ledit phényle substitué portant un groupement amino et/ou 1, 2 ou 3 atomes halogéno; et
dans laquelle X est 〉C=O, 〉CH-OH, 〉CH-O-C(O)-R⁹, 〉CH₂, 〉C(Oalkyle(C₁-₆))₂, 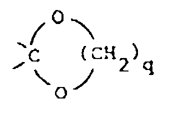 〉C=N-OH or 〉C=N-NH₂;
ledit R⁹ étant hydrogène ou alkyle(C₁₋₆) et
ledit q étant un entier égal à 2 ou 3; ou
R¹ est un radical de formule 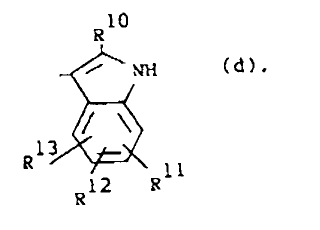 dans laquelle R¹⁰ est hydrogène ou alkyle(C₁₋₆); R¹¹, R¹², et
R¹³ sont chacun indépendamment hydrogène ou halogéno;
ou R¹ est un radical de formule 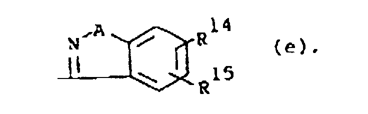 dans laquelle A est O ou S; R¹⁴ et R¹⁵ sont chacun indépendamment hydrogène, halogéno, hydroxy, alkyloxy(C₁₋₆) ou alkyle(C₁₋₆); caractérisé en ce que le principe actif est mélangé intimement avec le support, et si on le souhaite, avec d'autres principes actifs.

2. Procédé selon la revendication 1, caractérisé en ce que R¹ est un radical de formule (c).

3. Procédé selon la revendication 2, caractérisé en ce que Q est un radical de formule (a) dans laquelle Y¹ et Y² sont tous deux des atomes d'oxygène et R² et R³ sont tous deux hydrogène: ou en ce que Q est un radical de formule (b), dans laquelle R⁴ est méthyle, Z est -CR⁵=CR⁶ dans laquelle R⁵ et R⁶ sont indépendamment hydrogène ou méthyle, A est -CR⁷=CR⁸- dans laquelle R⁷ et R⁸ sont indépendamment hydrogène ou méthyle; et en ce que dans le radical (c) X est 〉C=O, et Ar est phényle susbstitué par halogéno.

4. Procédé selon la revendication 1, caractérisé en ce que le composé est la 3-[2-[4-(4-fluorobenzoyl)-1-pipéridinyl]éthyl-2,4-(1H, 3H-quinazolinedione ou la 3-[2-[4-(4-fluorobenzoyl)-1-pipéridinyl]éthyl-2,7-diméthyl-4H-pyrido[1,2-a]pyrimidin-4-one.

5. Procédé selon la revendication 5, caractérisé en ce que la composition est applicable par voie topique.

6. Procédé selon la revendication 5, caractérisé en ce que la composition est une poudre, une solution ou suspension aqueuse ou huileuse ou une formulation semi-liquide.

7. Procédé selon la revendication 6, caractérisé en ce que la composition est une crème, une gelée, une pâte, une pommade ou un baume.

8. Procédé de préparation d'une couverture de plaie contenant une composition de cicatrisation des plaies comme définie dans l'une quelconque des revendications 1 à 7, caractérisé en ce que ladite composition de cicatrisation des plaies est mise en contact avec la couverture de plaie.

9. Procédé selon la revendication 8, caractérisé en ce que la couverture de plaie est un plâtre, un bandage, un pansement ou un tampon de gaze imprégné ou arrosé d'une composition liquide de cicatrisation des plaies ou sur lequel on a répandu une composition solide pulvérulente de cicatrisation des plaies ou enduit, recouvert ou enrobé d'une composition de cicatrisation des plaies semi-liquide.
